## Europäisches Patentamt

## European Patent Office

## Office européen des brevets

(11) Veröffentlichungsnummer: **0 336 284**
**A2**

# (12) EUROPÄISCHE PATENTANMELDUNG

(21) Anmeldenummer: 89105580.8

(51) Int. Cl.⁴: **C12N 9/22**

(22) Anmeldetag: 30.03.89

(30) Priorität: 02.04.88 DE 3811277

(43) Veröffentlichungstag der Anmeldung:
11.10.89 Patentblatt 89/41

(84) Benannte Vertragsstaaten:
AT BE CH DE ES FR GB GR IT LI LU NL SE

(71) Anmelder: **BOEHRINGER MANNHEIM GMBH**
**Sandhofer Strasse 116**
**D-6800 Mannheim 31(DE)**

(72) Erfinder: **Bolton, Bryan John**
**7 Chalfont Court Brakcne Lane**
**Shirley Southampton(GB)**
Erfinder: **Jarsch, Michael, Dr.rer.nat.**
**Unterkarpfsee 11**
**D-8173 Bad Heilbrunn(DE)**
Erfinder: **Schmitz, Gudrun, Dr.rer.nat.**
**Wettersteinstrasse 3**
**D-8139 Bernried(DE)**
Erfinder: **Kessler, Christoph, Dr.rer.nat.**
**Fraunhoferstrasse 12**
**D-8000 München 5(DE)**

(54) **Typ II-Restriktionsendonuklease Ec1XI.**

(57) Die Restriktionsendonuklease EclXI besitzt folgende Erkennungssequenz und spaltet bevorzugt an der durch die Markierung definierten Spaltstelle:

$$5'\,C\,|\,GGCC\;\;G\text{-}3'$$
$$3'\,G\;\;CCGG\,|\,C\text{-}5'$$

Sie ist isoschizomer zu XmaIII, aus Mikroorganismen der Gattung Enterobacter erhältlich und kann zur Erkennung und Spaltung der komplementären doppelsträngigen DNA-Sequenz 5'-CCGCCG-3' verwendet werden.

EP 0 336 284 A2

## Typ II-Restriktionsendonuklease EclXI

Die Erfindung betrifft die neue Typ II-Restriktionsendonuklease ECIXI, ein Verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden Phosphodiesterbrücken in der Zielsequenz hydrolysiert und zwar in jedem Polynukleotidstrang eine. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch ist ihre Zugänglichkeit häufig unbefriedigend. In vielen Fällen sind die Mikroorganismen, aus denen diese Restriktionsendonukleasen isoliert werden, schwer zu kultivieren oder pathogen. Ebenso ist die Ausbeute bei großtechnischer Herstellung oft gering.

Die Restriktionsendonuklease XmaIII aus Xanthomonas malvaceanum (Gene 47 (1986) 1 - 153) kann frei von Xmal und Xma II nur in geringer Ausbeute und geringer Reinheit isoliert werden.

Der Erfindung liegt daher die Aufgabe zugrunde, ein Isoschizomeres zu XmaIII zu finden, welches einfacher zu isolieren ist und in höherer Ausbeute erhalten werden kann.

Gelöst wird diese Aufgabe erfindungsgemäß durch eine Restriktionsendonuklease mit der Erkennungssequenz und der durch die Markierung definierten Spaltstelle

$$5'-C \mid GGCC \ G-3'$$

$$3'-G \ CCGG \mid C-5'$$

welche dadurch gekennzeichnet ist, daß sie aus Mikroorganismen der Gattung Enterobacter erhältlich ist.

Die erfindungsgemäße Typ II-Restriktionsendonuklease, die im folgenden als EclXI bezeichnet wird, hat ein Temperaturoptimum bei ca. 37°C. Das Enzym besitzt eine gute Aktivität zwischen pH 7,2 und pH 7,8 bei einer Konzentration eines einwertigen Kations bei ca. 50 mmol/l (für NaCl) in 10 mmol/l MgCl$_2$ und 1 mmol/l Dithioerythritol (DTE). Das pH-Optimum liegt bei ca. pH 7,5. EclXI ist ein Isoschizomer zu XmalII. Das pH-Optimum von XmaIII liegt zwischen pH 8,2 und pH 9,0. In Gegenwart von NaCl wird XmaIII inhibiert.

Die Erkennungssequenz läßt sich durch vollständigen Verdau der DNAs der Viren SV 40 und Adeno 2, der Phagen Lambda, phiX174, den Phagenderivaten M13mp8 und den Plasmiden pBR322 und pBR328 bestätigen. Diese DNA-Moleküle werden mit EclXI behandelt.

Tabelle I zeigt einen Vergleich der experimentell beobachteten Schnittstellenspezifität mit einer computerermittelten Schnitt stellenspezifität für ein Enzym, welches die Sequenz CGGCCG erkennt.

TABELLE I

| DNA | Anzahl der experimentell bestimmten Schnittstellen | Anzahl der durch Computeranalyse bestimmten Schnittstellen | Experimentell bestimmte Fragmentlängen | Durch Computeranalyse bestimmte Fragmentlängen | Spaltpositionen ermittelt durch Computeranalysen |
|---|---|---|---|---|---|
| SV 40 | 0 | 0 | 0 | 0 | 0 |
| phiX174 | 0 | 0 | 0 | 0 | 0 |
| M13mp8 | 0 | 0 | 0 | 0 | |
| pBR322 | 1 | 1 | 4300 bp | 4363 bp | bei 939 bp |
| pBR328 | 2 | 2 | 3850 bp | 3859 bp | bei 908 bp |
| | | | 1050 bp | 1048 bp | und 4767 bp |
| bp: Basenpaar(e) | | | | | |

Die Schnittstelle innerhalb der Erkennungssequenz des Enzyms läßt sich wie folgt feststellen:

Das Plasmid pBR328 wird mit ClaI linearisiert. Das EcoRI-Fragment wird am 5-Ende mit T4-Polynucleotid-Kinase unter Verwendung von [$\gamma^{32}$P]-ATP markiert und am 3'-Ende mit terminaler Transferase und [$\alpha^{32}$P]-ddATP $^{32}$P-markiert. Nach einem weiteren Verdau werden die entstandenen Fragmente, welche am ClaI-Ende 5'-markiert oder 3'-markiert sind, durch Agarose-gel-Elektrophorese isoliert und aus dem Gel gereinigt.

Aliquote dieser Fragmente werden mit EclXI behandelt und gleichzeitig einer basenspezifischen chemischen Spaltung (Maxam and Gilbert, Methods in Enzymology 65, 1980, 499-560) zur Sequenzierung unterworfen.

Die Analyse der Fragmente wird durch Sequenzgel Elektrophorese (6 % Polyacrylamid, 8 mol/l Harnstoff) und anschließende Autoradiographie durchgeführt. Die Interpretation der Ergebnisse wird analog Methods in Enzymology 65, 1980, 391-401 durchgeführt. Hierbei zeigt sich, daß EclXI zwischen Position 4767 und 4768 (Basenpaare) in der Sequenz des Vektors pBR328 mit folgender Spezifität schneidet:

$$5'-GGC | GGCC-GAC-3'$$
$$3'-CCG \quad CCGG | CTG-5'$$

Damit ergibt sich als allgemeine Spezifität:

$$5'-C | GGCC \quad G-3'$$
$$3'-G \quad CCGG | C-5'$$

Die experimentell bestimmte Anzahl der Schnittstellen ist identisch mit der Anzahl der Schnittstellen, die durch Computeranalyse mit den verschiedenen DNAs für die Sequenz CGGCCG erhalten wurde (Tabelle 1). Zusätzlich wurden diese Daten noch mit den Tabellen aus Gene 10 (1980) 357 - 370, verglichen.

Ein doppelter Verdau von pBR322 DNA mit EcoRI und EclXI sowie von pBR328 DNA mit PstI und EclXI bestätigen, daß die Spezifität CGGCCG ist.

Erfindungsgemäß wird EclXI gewonnen, indem man Microorganismen der Gattung Enterobacter züchtet und das Enzym aus den Zellen gewinnt. Vorzugsweise werden Mikroorganismen der Art Enterobacter cloacae, besonders bevorzugt der Stamm Enterobacter cloacae DSM 4319 verwendet.

Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise λDNA. Die erhaltenen DNA-Fragmente werden in Agarosegelen in den für die Fragmentauftrennung üblichen Puffersystemen in Gegenwart von Ethidiumbromid elektrophoretisch aufgetrennt.

Der für die Gewinnung des Enzyms vorzugsweise verwendete Mikroorganismus Enterobacter cloacae, DSM 4319, wächst aerob in Merck Standard I Medium.

Der Mikroorganismus wurde bei der Deutschen Sammlung von Mikroorganismen, Gesellschaft für biotechnologische Forschung mbH, Mascheroder Weg 16, 3300 Braunschweig, BRD, hinterlegt und trägt die Hinterlegungsnummer DSM 4319. Die optimalen Wachstumsbedingungen sind bei 10 - 30° C pH 7,2 - 7,8. Die Verdoppelungszeit beträgt etwa 1 Stunde.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen einem Überdruck von 5 bar ausgesetzt. Die hierbei entstandene Zellenmasse wird in Tris-HCl Puffer pH 8,0, welcher Protease-Inhibitoren enthält, resuspendiert. Anschließend werden die Zellen über eine French-Presse aufgeschlossen und mit Polymin und Ammoniumsulfat präzipitiert. Die weitere Reinigung des Überstands wird vorzugsweise über Molekularsiebchromatographie, Ionentauscherchromatographie sowie Affinitätschromatographie durchgeführt. Ein geeignetes Molekularsieb ist beispielsweise Ultrogel AcA34 (LKB).

4

Als Anionentauscher geeignet ist das unter dem Namen DEAE Sephadex (Pharmacia) erhältliche Produkt. Als Material für die Affinitätschromatographie geeignet ist beispielsweise Heparin-Sepharose CL-6B (Pharmacia). Aber auch andere Chromatographiematerialien, die dem Fachmann bekannt sind, sind geeignet. Die folgenden Beispiele erläutern die Erfindung weiter.

Beispiel 1:

Enterobacter cloacae DSM 4319 wird bei 30° C 10 Stunden gezüchtet und der späten logarithmischen bzw. stationären Phase geerntet. Als Kulturmedium wird Merck Standardmedium I verwendet.

Die Zellpaste (30 g Naßgewicht) wird in 3 Volumen Puffer A (40 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 23.000 lb/inch$^2$ aufgeschlossen. Anschließend wird Polymin P zugegeben und der Niederschlag abgetrennt. Der Überstand wird anschließend durch Zugabe von 90 % (w/v) Ammoniumsulfat behandelt. Der hierbei entstandene Niederschlag wird in 15 ml Puffer B (40 mmol/l Tris-HCl, pH 8,0, 0,1 mmol/l EDTA, 7 mmol/l 2-Mercaptoethanol, 10 (v/v) Glycerin) aufgelöst. Anschließend wird nach Dialyse gegen Puffer B an einer Heparin-Sepharose-Säule fraktioniert. Zur Elution wird ein Gradient von 0 - 1 mol/l NaCl verwendet. EclXI wird in der Fraktion zwischen 0,5 und 0,6 mol/l NaCl gefunden. Die aktiven Fraktionen werden mit 90 % Ammoniumsulfat gefällt, der Niederschlag in Puffer B aufgenommen und nach Dialyse gegen Puffer B an einer Ultragel AcA34-Säule fraktioniert, welche zuvor mit Puffer B, dem 0,5 mmol/l NaCl zugesetzt wird, equilibriert ist. Die aktive Fraktion wird gegen Puffer B dialysiert. Anschließend wird sie auf eine Phosphocellulose-Säule, welche mit Puffer B equilibriert wurde, aufgegeben. Zur Elution wird ein Gradient von 0 - 1,0 mmol/l NaCl in Puffer B verwendet. EclXI wird in der Fraktion zwischen 0,3 und 0,4 mmol/l NaCl gefunden. Die aktive Fraktion wird gegen Puffer B dialysiert und auf eine DEAE-Sepharose-Säule, die mit Puffer B equilibriert wurde, aufgebracht. Zur Elution wird ein Gradient von 0 - 1,0 mmol/l NaCl in Puffer B angewendet. EclXI wird in der Fraktion, die zwischen 0,05 und 0,15 mmol/l NaCl erscheint, gefunden.

Die aktiven Fraktionen werden zusammengegeben und gegen Lagerpuffer (20 mmol/l Tris-HCl, pH 8,0, 10 mmol/l 2-Mercaptoethanol, und 100 mmol/l NaCl, 50 % (v/v) Glycerin) dialysiert.

Beispiel 2

Bestimmung der Aktivität

Definition der Enzymeinheiten:

1 U EclXI spaltet 1 μg Lambda-DNA innerhalb 1 Stunde bei 37° C in 25 μl Endvolumen.

Zu einer Mischung von 12 μl Inkubationspuffer (10 mmol/l Tris-HCl, pH 7,5/37° C, 10 mmol/l Magnesiumchlorid, 50 mmol/l NaCl, und 1 mmol/l DTE werden 7 μl Wasser und 5 μl Lambda DNA (optische Dichte: 4 OD/ml) sowie 1 μl EclXI-Lösung (1U/μl) zugegeben. Die Lösung wird eine Stunde bei 37° C inkubiert, auf Eis gekühlt und mit 5 μl eines Abstopp-Reagenses, bestehend aus 7 mmol/l Harnstoff, 20 % (w/v) Saccharose, 60 mmol/l EDTA und 0,01 % (w/v) Bromphenolblau versetzt. Anschließend wird eine Trennung durch Elektrophorese in 1 % Agarose-Gelen für 16 Stunden bei 20 V durchgeführt. Die erhaltenen Banden werden über Vergleich mit einem DNA-Längenstandard identifiziert.

**Ansprüche**

1. Typ II-Restriktionsendonuklease, mit der Erkennungssequenz und der durch die Markierung definierten Spaltungsstelle

```
5'C|GGCC G-3'
    └──────┐
3'G  CCGG|C-5'
```

dadurch gekennzeichnet, daß sie ein pH-Optimum zwischen pH 7,2 und pH 7,8 aufweist und aus einem Mikroorganismus der Gattung Enterobacter erhältlich ist.

2. Typ II-Restriktionsendonuklease nach Anspruch 1, dadurch gekennzeichnet, daß sie aus einem Mikroorganismus der Art Enterobacter cloacae erhältlich ist

3. Typ II-Restriktionsendonuklease nach Anspruch 2, dadurch gekennzeichnet, daß sie aus Enterobacter cloacae DSM 4319 erhältlich ist.

4. Restriktionsendonuklease nach Anspruch 1 bis 3, gekennzeichnet durch ein Temperatur-Optimum bei ca. 37° C.

5. Verfahren zur Gewinnung einer Typ II-Restriktionsendonuklease mit der die Erkennungssequenz und der durch die Markierung definierten Spaltungsstelle

```
5'C|GGCC G-3'
    └──────┐
3'G  CCGG|C-5'
```

dadurch gekennzeichnet, daß man einen Mikroorganismus der Gattung Enterobacter züchtet und das Enzym aus den Zellen gewinnt.

6. Verfahren zur Gewinnung einer Restriktionsendonuklease nach Anspruch 5 dadurch gekennzeichnet, daß man einen Mikroorganismus der Art Enterobacter cloacae züchtet und das Enzym aus den Zellen gewinnt.

7. Verfahren nach Anspruch 6, dadurch gekennzeichnet, daß man als Mikroorganismus Enterobacter cloacae DSM 4319 verwendet.

8. Verfahren nach Anspruch 5 bis 7, dadurch gekennzeichnet, daß man die Zellen aufschließt und den Zellüberstand gewinnt.

9. Verfahren nach Anspruch 8, dadurch gekennzeichnet, daß man zur Feinreinigung den Zellüberstand einer Affinitätschromatographie, einer Molekularsiebchromatographie und einer abschließenden Chromatographie über einen Kationentauscher unterwirft.

10. Verfahren nach Anspruch 9, dadurch gekennzeichnet, daß man zur Affinitätschromatographie trägerfixiertes Heparin verwendet.

11. Verwendung einer TypII-Restriktionsendonuklease, mit der Erkennungssequenz und der durch die Markierung definierten Spaltungsstelle

```
5'C|GGCC G-3'
    └──────┐
3'G  CCGG|C-5'
```

die ein pH-Optimum zwischen pH 7,2 und pH 7,8 aufweist und aus einem Mikroorganismus der Gattung Enterobacter erhältlich ist, zur Erkennung und Spaltung der komplementären doppelsträngigen DNA-Sequenz
5'-CGGCCG-3'
3'-GCCGGC-5'